# EUROPEAN PATENT APPLICATION

(11) **EP 4 717 166 A1**
(43) Date of publication of application: **01.04.2026**
(21) Application number: 24203221.7
(22) Date of filing: 27.09.2024
(51) Int. Cl.: A61B 5/145

(54) **TRANSMITTER MODULE, HUMAN BODY INDICATOR MONITORING DEVICE AND METHOD OF USING THE SAME**

(71) Applicant: Syai UK Ltd, Cambridge CB2 8DL (GB)
(72) Inventor: Wang, Zhihua, Cambridge, CB2 8DL (GB)
(74) Representative: Nederlandsch Octrooibureau

(57) **Abstract**

The present invention relates to the technical field of medical equipment, and disclosed thereby are a transmitter module, a human body indicator monitoring device, and a method of using the same. The transmitter module comprises a transmitter bottom shell, a casing, a needle, and a sensor. The transmitter bottom shell has an avoidance through-hole; the casing is hermetically connected to an outer surface of the transmitter bottom shell, and the casing surrounds the avoidance through-hole; the needle comprises a needle body and a needle handle connected to one end of the needle body; the needle handle is located on the side, away from the casing, of the transmitter bottom shell and is hermetically connected to the transmitter bottom shell to block the avoidance through-hole; the needle body runs through the avoidance through-hole, and the end away from the needle handle is located inside the casing, with the sensor inserted into the needle body. The needle handle is located on the side, away from the casing, of the transmitter bottom shell without any part exposed on the bottom side of the transmitter bottom shell, so there is no need for extra needle return action. After the needle injection is completed, only one needle return is required.

## Description

### TECHNICAL FIELD

The present invention relates to the technical field of medical equipment, and particularly to a transmitter module, a human body indicator monitoring device, and a method of using the same.

### BACKGROUND

For diseases requiring long-term monitoring of specific human body indicators, such as diabetes, a device that may continuously monitor human body indicators is needed to help patients complete data collection and analysis in real time. At present, such a device has a complex structure and is not easy to use.

### SUMMARY

The human body indicator detection device generally includes four parts: sensor: used to be implanted in the human body, and convert chemical reactions into electrical signals; signal transmitter: used to transmit electronic signals collected by a sensor to devices (such as mobile phones) through wireless signals; sensor guide pin: used to guide the sensor (which may be soft) into a needle (such as a hard metal needle), insert the needle with the sensor into the human body, and then pull out (return) the needle, leaving the sensor in the human body; sealing component: as the sensor and needle need to be inserted into the human body, it is necessary to ensure that these two components are sterilized before insertion to avoid infection. The plastic needle handle should be threaded through the hole in the middle of the transmitter and then screwed together with the sealing casing on the other side.

In some cases, the sealing structure of the sensor and needle requires the plastic needle handle to pass through the lower cover of the transmitter and expose enough length for the casing for fastening. Therefore, when the user uses it, after removing the casing and before injecting the needle into the human body, the needle should return to retract the plastic handle that exposes the bottom cover of the transmitter to avoid touching the human body and obstructing the transmitter from sticking to the body surface. As a result, the operation of the insertion device module is complex. The insertion device module refers to the component used to inject the needle and sensor of the transmitter module along the needle injection direction, so that the needle with the sensor may be injected into the human body, and the needle may be returned and pulled out of the human body.

The present invention discloses a transmitter module, a human body indicator monitoring device and a method of using the same, used to achieve sealing of the needle body and the sensor while avoiding unnecessary return. "Return" herein refers to the return action of the needle.

To achieve the above purpose, the present invention provides the following technical solutions:
In a first aspect, a transmitter module is provided. The transmitter module comprises: a transmitter bottom shell, a casing, a needle, and a sensor. The transmitter bottom shell has an avoidance through-hole; the casing is hermetically connected to an outer surface of the transmitter bottom shell, and the casing surrounds the avoidance through-hole; the needle comprises a needle body and a needle handle connected to one end of the needle body; the needle handle is located on the side of the transmitter bottom shell away from the casing and is hermetically connected to the transmitter bottom shell to block the avoidance through-hole; the needle body runs through the avoidance through-hole, and the end away from the needle handle is located inside the casing, with the sensor inserted into the needle body.

The needle handle is located on the side, away from the casing, of the transmitter bottom shell without any part exposed on the bottom side of the transmitter bottom shell, so there is no need for extra needle return action. After the needle injection is completed, only one needle return is required. The sterile sealing of the needle body and sensor is achieved through an enclosed space formed by the hermetic connection between the casing and the outer surface of the transmitter bottom shell, as well as the hermetic connection between the needle handle and the transmitter bottom shell.

Optionally, the end of the casing facing the transmitter bottom shell has an annular connection portion and an annular support platform; the annular connection portion is fixedly connected to the transmitter bottom shell, and a first sealing rubber ring is provided between the annular support platform and the transmitter bottom shell.

Optionally, the annular connection portion and the transmitter bottom shell are fixed by ultrasonic welding or glue.

Optionally, the transmitter module further comprises a sealing bracket located between the transmitter bottom shell and the needle handle; the sealing bracket comprises an annular support plate and an annular protrusion connected to one side surface of the annular support plate, and the transmitter bottom shell has an annular groove surrounding the avoidance through-hole; the annular protrusion is located inside the annular groove, and the annular groove is filled with sealant; the annular support plate overlaps with the transmitter bottom shell.

Optionally, the transmitter bottom shell has an annular boss surrounding the avoidance through-hole, and the annular groove is provided on the annular boss; the sensor comprises a vertical portion located inside the needle body and a horizontal portion connected to one end of the vertical portion; the annular boss is provided with a first slit, and the annular protrusion is provided with a second slit corresponding to the first slit; the horizontal portion is located on the transmitter bottom shell, and runs through the first slit and the second slit; the first slit and the second slit are filled with the sealant.

Optionally, the needle handle has a stepped surface, and a second sealing rubber ring is provided between the stepped surface and the annular boss.

Optionally, the needle handle, the sealing bracket, the transmitter bottom shell and the casing are all made of transparent materials.

Optionally, the transmitter module further comprises a transmitter top shell, and the transmitter top shell is located on the side of the transmitter bottom shell away from the casing, and encloses an accommodating space with the transmitter bottom shell; a circuit board and a connector are further provided in the accommodating space, and the sensor is connected to the circuit board through the connector; the circuit board comprises a first board portion and a second board portion; one end of the first board portion and one end of the second board portion are bent and connected by a connection portion; the first board portion is located between the second board portion and the transmitter top shell; the end of the first board portion away from the connecting portion is abutted to the surface of the annular support plate facing the transmitter bottom shell.

Optionally, the needle body is in contact with the inner wall of the avoidance through-hole.

Optionally, the outer surface of the transmitter bottom shell is provided with adhesive.

In a second aspect, a human body indicator monitoring device is provided. The human body indicator monitoring device comprises an insertion device module and the transmitter module as mentioned in any of the above technical solutions, wherein the transmitter module is installed on the insertion device module, and the insertion device module is used to inject a needle and sensor of the transmitter module into a human body, and used to return the needle.

Compared with the prior art, the advantages of the human body indicator monitoring device and the transmitter module mentioned above are the same, and will not be repeated here.

In a third aspect, a method of using the human body indicator monitoring device as mentioned in the above technical solutions is provided. The insertion device module comprises: a base support, a main bracket, a needle bracket, a first elastic reset member and a second elastic reset member, wherein the main bracket comprises a base and a first locking member connected to the base;

The method comprises:
taking the insertion device module closer to the human body;
releasing the limit between the main bracket and the base support,
wherein the first elastic reset member drives the main bracket to move from an initial station to an injection station via an unlocking station along an injection direction; when the main bracket is located at the initial station, the first locking member is clamped between the needle bracket and the base support, and locked to the needle bracket; when the main bracket moves to the unlocking station, at least part of the first locking member is located inside an avoidance groove of the base support to unlock the needle bracket;
the first elastic reset member continues to drive the main bracket to move to the injection station, so that the needle and the sensor are injected into a human body, and the second elastic reset member drives the needle bracket to return from an original station to a return station along a return direction, so as to pull the needle out of the human body, wherein the return direction is opposite to the injection direction; and
removing the insertion device module.

Optionally, after pulling out the needle from the human body and leaving the sensor inside the human body, the method further comprises: the sensor is in signal connection to a transmitter, and a signal collected by the sensor is transmitted to an additional device via the transmitter in a wireless manner; or, the sensor is in direct signal connection to the additional device, or, the transmitter is in signal connection to the sensor, and a signal collected by the sensor is transmitted to the additional device via the transmitter.

Compared with the prior art, the advantages of the method and the human body indicator monitoring device mentioned above are the same, and will not be repeated here.

### BRIEF DESCRIPTION OF DRAWINGS

FIG.1 shows a schematic diagram of the overall structure of the human body indicator monitoring device provided in the embodiment of the present application;
FIG.2 shows an exploded view of the main body a in FIG. 1;
FIG.3a shows a schematic diagram of the structure as shown in FIG.2 assembled with the main bracket 205, the first elastic reset member 206 and the base support 207;
FIG.3b shows a structural diagram of the main bracket 205 in FIG.3a;
FIG.4 shows a schematic diagram of the structure as shown in FIG.3a installed with a needle bracket 204 and a second elastic reset member 203;
FIG.5 shows a schematic diagram of the structure as shown in FIG.4 installed with a first subshell 2081, a second subshell 2082 and a decorative ring 201;
FIG.6 shows a schematic diagram of the structure as shown in FIG.5 installed with an injection switch 210 and a third elastic reset member 209;
FIG.7 shows a schematic diagram of the external structure of the structure as shown in FIG.6;
FIG.8 shows a schematic diagram of the structure as shown in FIG.1 assembled with the transmitter bottom shell 121 and the casing 110 of the transmitter module 100;
FIG.9 shows a schematic diagram of the structure as shown in FIG.8 assembled with the sensor 130, the sealing bracket 140 and the needle 150;
FIG.10a shows an assembly diagram corresponding to FIG.9;
FIG.10b shows a partial enlarged view of the structure as shown in FIG.10a;
FIG.10c shows a structural diagram of the sealing bracket 140 in FIG.10b;
FIG.10d shows a structural diagram of the transmitter bottom shell 121 in FIG.10b;
FIG. 11 shows a schematic diagram of irradiation sterilization on the structure as shown in FIG.10a;
FIG.12 shows an exploded view of the structure as shown in FIG.11 installed with the transmitter top shell 122 and the circuit board 123;
FIG.13 shows an exploded view of the assembly structure installed with the release paper and adhesive in the exploded view as shown in FIG.12;
FIG.14 shows an assembly diagram corresponding to FIG.13;
FIG.15 shows a schematic diagram before installing the transmitter module 100 as shown in FIG.14 into the main body a;
FIG.16 shows a schematic diagram after installing the transmitter module 100 as shown in FIG.14 into the main body a;
FIG.17 shows a sectional view of the structure as shown in FIG.1 after fixing the bottom shell b to the bottom side of the main body a;
FIG.18 shows a schematic diagram of the appearance of the human body indicator monitoring device corresponding to FIG.17;
FIG.19 shows a schematic diagram of the human body indicator monitoring device provided in the embodiment of the present application with the bottom shell b unscrewed;
FIG.20 shows a schematic diagram of the structure as shown in FIG.19 with the bottom shell b removed;
FIG.21 shows a schematic diagram of the structure as shown in FIG.20 after breaking the anti-touch handle 2101;
FIG.22 shows a state diagram of the sensor 130 and the transmitter N in FIG.21 when working in a human body M1.

Callouts: 100-transmitter module; 110-casing; 1101-ratchet tooth; 111-annular connection portion; 112-first sealing rubber ring; 113-annular support platform; 121-transmitter bottom shell; 1211-annular boss; 122-transmitter top shell; 123-circuit board; 1231-first board portion; 1232-second board portion; 1233-connection portion; 124-battery; 125-connector; 130-sensor; 131-vertical portion; 132-horizontal portion; 140-sealing bracket; 141-annular protrusion; 142-annular support plate; 143-annular fence; 144-sealant; 150-needle; 151-needle handle; 152-needle body; 153-second sealing rubber ring; 161-adhesive; 162-release paper; 200-insertion device module; 201-decorative ring; 202-transmitter bracket; 203-second elastic reset member; 204-needle bracket; 2041-inner cylinder wall; 2042-outer cylinder wall; 2043-hoop; 205-main bracket; 2051-base; 2052-second locking member (or second buckle); 2053-third buckle; 2055-first locking member (or first buckle); 206-first elastic reset member; 207-base support; 2071-inner cylinder; 2072-outer cylinder; 2081-first subshell; 2082-second subshell; 209-third elastic reset member; 210-injection switch; 2101-anti-touch handle; 221-shell body; 222-middle support skeleton; 224-elastic buckle; a-main body; b-bottom shell; F-annular groove; G1-first slit; G2-second slit; K-clamping hole; M1-human body; N-transmitter; P-axial direction; P1-injection direction; P2-return direction; R-stepped surface; S1-horizontal clamping surface; S2-inclined guide surface; S3-clamping positioning surface; S4-inclined blocking surface; SS-inclined chamfered surface; S6-outer surface; T1-needle handle via hole; U-avoidance groove; V-clamping slot; W-avoidance through-hole.

### DETAILED DESCRIPTION OF THE EMBODIMENTS

Technical solutions in the embodiments of the present invention will be described clearly and completely in combination with figures in the embodiments of the invention. Obviously, the described embodiments are only part, but not all, of the embodiments of the invention. All other embodiments obtained by those of ordinary skill in the art without creative work based on the embodiments of the present invention are within the scope of protection of the present invention.

The human body indicator monitoring device provided by the embodiments of the present application may specifically be a glucometer, which is used to help diabetics monitor the changes in blood glucose values for a long time. However, it is not limited to glucometers, but may also be equipment for collecting and analyzing other specific body indicators.

In order to have a comprehensive understanding of the human body indicator monitoring, a preliminary introduction will be given below to the human body indicator monitoring device provided in the embodiments of the present application.

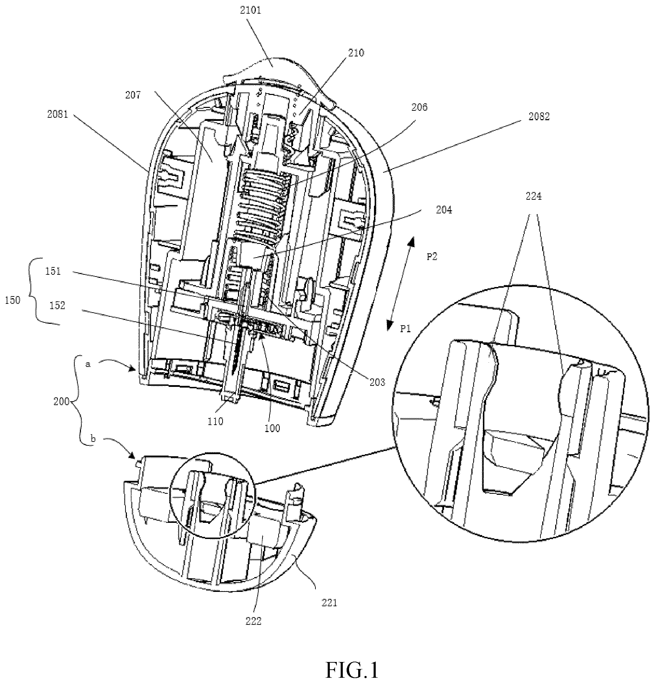

FIG.1 shows a schematic diagram of the overall structure of the human body indicator monitoring device provided in the embodiment of the present application. Referring to FIG.1, the human body indicator monitoring device provided in the embodiment of the present application comprises a transmitter module 100 and an insertion device module 200. The insertion device module 200 may comprise a main body a and a bottom shell b. The transmitter module 100 may be installed inside the bottom side of the main body a, while the bottom shell b may be fixed to the bottom side of the main body a to provide physical protection for the transmitter module 100. The main body a of the insertion device module 200 is mainly used to inject the needle 150 (such as a hard metal needle) and the sensor 130 of the transmitter module 100 along the injection direction P1, inject the needle 150 with the sensor 130 into the human body M1, and return the needle 150 along the return direction P2, pull the needle out of the human body M1, and leave the sensor inside the human body M1. Then the transmitter may be in signal connection to the sensor, and may transmit the signal collected by the sensor to an additional device (such as mobile phones) in a wireless manner, where the return direction P2 is opposite to the injection direction P1. The sensor may be in signal connection to the transmitter after being left in the human body M1, but may also be in direct signal connection to an additional device (such as mobile phones). This optional feature may be applied throughout the embodiments of the invention and the description.

Its structure will be illustrated below with the assembly process of the main body a:

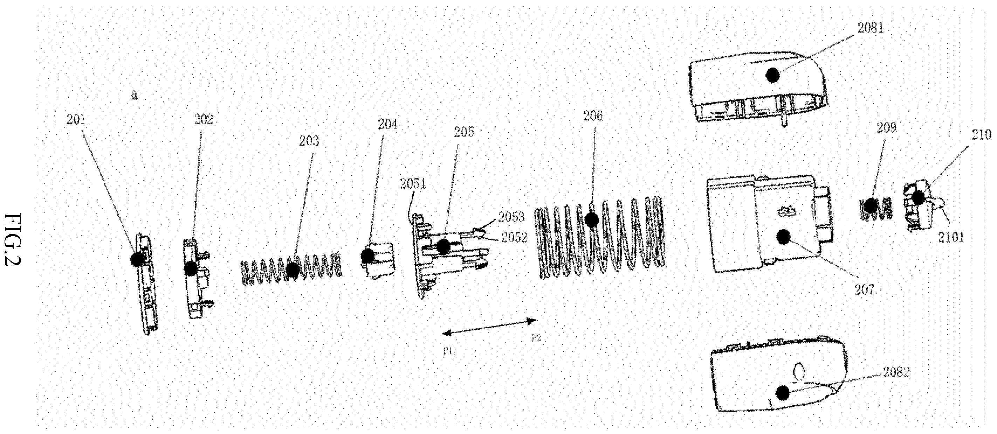

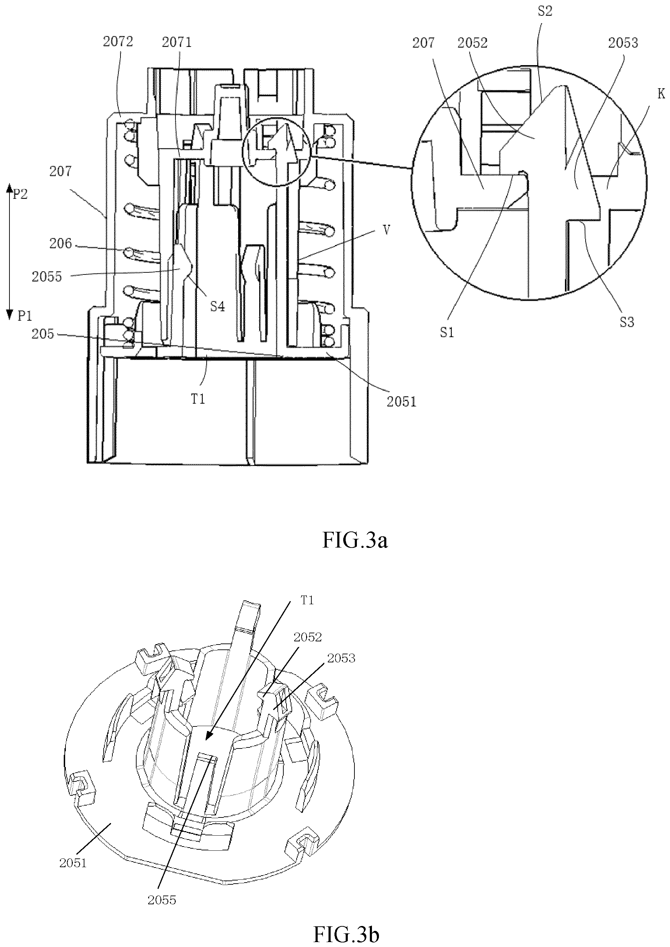

FIG.2 shows an exploded view of the main body a in FIG.1, FIG.3a shows a schematic diagram of the structure as shown in FIG.2 assembled with the main bracket 205, the first elastic reset member 206 and the base support 207, and FIG.3b shows a structural diagram of the main bracket 205 in FIG.3a; combining FIG.2, FIG.3a and FIG.3b, the base support 207 may comprise a main sleeve with an opening facing the injection direction P1. The main sleeve may comprise an inner cylinder 2071 and an outer cylinder 2072 which are coaxial, and the bottom of the inner cylinder 2071 may have a certain depression relative to the bottom of the outer cylinder 2072 along the injection direction P1.

The main bracket 205 may comprise a base 2051 and a first locking member 2055 and a second locking member 2052 connected on the base 2051, wherein the first locking member 2055 and the second locking member 2052 may be in different forms, for example, the first locking member 2055 may be a first buckle (with the same reference numeral of 2055), and the second locking member 2052 may be a second buckle (with the same reference numeral of 2052).

The first elastic reset member 206 may specifically be a coil spring and may be sleeved between the inner cylinder 2071 and the outer cylinder 2072. The depression portion at the bottom of the inner cylinder 2071 and the wall of the outer cylinder 2072 may form a limit on the corresponding end of the first elastic reset member 206, and the other end of the first elastic reset member 206 may be abutted to the base 2051.

The main bracket 205 may have an initial station, an unlocking station, and an injection station along the injection direction P1. The bottom of the main sleeve (specifically, the bottom of the inner cylinder 2071) may have a clamping hole K corresponding to the second buckle 2052. The second buckle 2052 may be clamped in the above-mentioned clamping hole K to compress the first elastic reset member 206. At this time, the first elastic reset member 206 may store elastic potential energy. The main bracket 205 is at the initial station, and the first elastic reset member 206 may be used to provide the first elastic reset force for the main bracket 205 to recover from the initial station to the injection station along the injection direction P1. The first elastic reset force keeps the main bracket 205 in a tendency to recover from the initial station to the injection station. Moreover, the first elastic reset member 206 may provide uniform elastic support for the main bracket 205 and the main sleeve at all positions in the circumferential direction by using the coil spring. However, it should also be understood that the first elastic reset member 206 is not limited to a coil spring, as long as it may provide the first elastic reset force for the main bracket 205 to recover from the initial station to the injection station.

Specifically, the second buckle 2052 has an inclined guide surface S2 and a horizontal clamping surface S1 arranged oppositely along the injection direction P1, and the horizontal clamping surface S1 may be clamped to the bottom of the inner cylinder 2071. However, the second locking member 2052 is not limited to the form of the second buckle 2052. As long as the main bracket 205 is located at the initial station, the second locking member 2052 and the base support 207 may be locked, and the locking form may be in various other forms.

The base 2051 may have a needle handle via hole T1 (referring to FIG.3b), through which the needle handle 151 of the needle 150 may pass. Specifically, the base 2051 may be circular, triangular, or other shapes, with the middle hole serving as the needle handle via hole T1.

There may be multiple second buckles 2052, and multiple second buckles 2052 are arranged around the needle handle via hole T1 to achieve more stable locking of the main bracket 205 and the base support 207 in the circumferential direction. There may also be multiple clamping holes K, and they may be arranged one-to-one with multiple second buckles 2052; each second buckle 2052 is clamped to a corresponding clamping hole K.

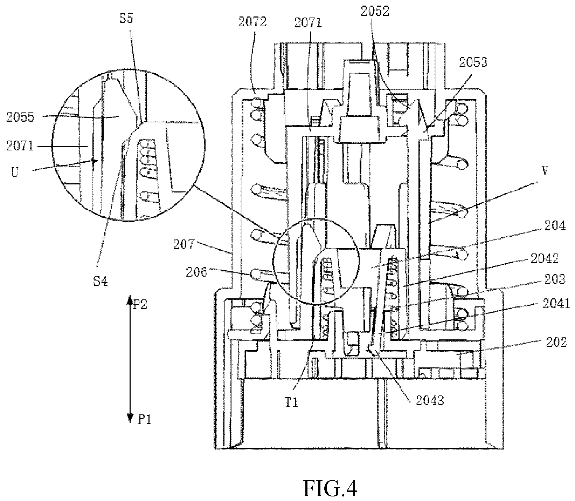

FIG.4 shows a schematic diagram of the structure as shown in FIG.3a installed with a needle bracket 204 and a second elastic reset member 203. Referring to FIG.4, the needle bracket 204 may comprise an inner cylinder wall 2041 and an outer cylinder wall 2042, and the top of the inner cylinder wall 2041 may be connected together with the top of the outer cylinder wall 2042; the bottom of the inner cylinder wall 2041 and the bottom of the outer cylinder wall 2042 may form an opening, and the bottom of the inner cylinder wall 2041 may form a hoop 2043, used to clamp the needle handle 151 of the needle 150.

In FIG.4, the needle bracket 204 is located in the needle handle via hole T1, and the main bracket 205 may be located at the initial station; the first buckle 2055 may be located inside the main sleeve, specifically inside the inner cylinder 2071 of the main sleeve. The first buckle 2055 is clamped with the needle bracket 204, limiting the needle bracket 204 in the return direction P2 to prevent the needle bracket 204 from moving along the return direction P2 and achieving the locking between the first buckle 2055 and the needle bracket 204. Moreover, the first buckle 2055 may be clamped between the needle bracket 204 and the base support 207, and the first buckle 2055 is limited by the base support 207 and the needle bracket 204 to prevent it being detached from the needle bracket 204 and unlocking the needle bracket 204. The first locking member 2055 is not limited to the form of the first buckle 2055. As long as the main bracket 205 is located at the initial station, the first locking member clip 2055 is provided between the needle bracket 204 and the base support 207, and locked with the needle bracket 204.

Specifically, referring to FIG.4, the first buckle 2055 may have an inclined blocking surface S4, and the edge of one end of the needle bracket 204 in the return direction P2 may be formed with an inclined chamfered surface S5. When the main bracket 205 is located at the initial station, the inclined blocking surface S4 of the first buckle 2055 is abutted to the inclined chamfered surface S5.

Once the first buckle 2055 is unlocked, the needle bracket 204 may move towards the return station under the action of the second elastic reset member 203, and the inclined blocking surface S4 is pushed along the return direction P2 by using the inclined chamfered surface S5. Due to the guiding effect of the inclined blocking surface S4 and the inclined chamfered surface S5, the needle bracket 204 may push off the first buckle 2055 laterally by using the inclined chamfered surface S5, detaching the first buckle 2055 from limiting the needle bracket 204 in the return direction P2.

When the base 2051 has the above-mentioned needle handle via hole T1, there may also be multiple first buckles 2055, and multiple first buckles 2055 may be uniformly spaced around the needle handle via hole T1 to achieve uniform and stable limit of the needle bracket 204 in the circumferential direction.

When there are multiple first buckles 2055 and second buckles 2052, the number may be the same, for example, three; multiple first buckles 2055 and multiple second buckles 2052 may be alternated in order, so that the first buckles 2055 and the second buckles 2052 may be distributed more uniformly.

The needle bracket 204 may have an original station and a return station relative to the main bracket 205 along the return direction P2. The base 2051 may be provided with a transmitter bracket 202 in the injection direction P1, and the transmitter bracket 202 may be clamped (for example, through a buckle) to the base 2051. The second elastic reset member 203 may specifically be a coil spring; one end may be abutted to the included angle formed by the top of the inner cylinder wall 2041 and the top of the outer cylinder wall 2042, and the other end may be abutted to the transmitter bracket 202; the second elastic reset member 203 is compressed by using the transmitter bracket 202. At this time, the needle bracket 204 may be located at the original station relative to the main bracket 205, and the second elastic reset member 203 may store elastic potential energy, to provide the second elastic reset force for the needle bracket 204 to recover from the original station to the return station along the injection direction P1. The second elastic reset force keeps the needle bracket 204 in a tendency to recover from the original station to the return station. Moreover, the second elastic reset member 203 may provide uniform elastic support for the needle bracket 204 and the transmitter bracket 202 at all positions in the circumferential direction by using the coil spring. However, it should also be understood that the second elastic reset member 203 is not limited to a coil spring, as long as it may provide the second elastic reset force for the needle bracket 204 to recover from the original station to the return station.

The base support 207 may have an avoidance groove U, which may be formed on the inner wall of the main sleeve, specifically the inner wall of the inner cylinder 2071. But the base support 207 is in different forms, the avoidance groove U may also be located at other positions of the base support 207. When the main bracket 205 is located at the unlocking station mentioned above, at least part of the first locking member 2055 may be located in the above-mentioned avoidance groove U. At this time, the first locking member 2055 releases the locking of the needle bracket 204.

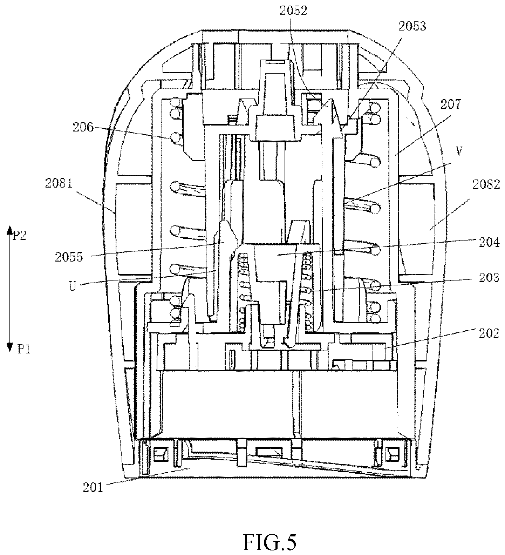

FIG.5 shows a schematic diagram of the structure as shown in FIG.4 installed with a first subshell 2081, a second subshell 2082 and a decorative ring 201. Referring to FIG.5, the first subshell 2081 and the second subshell 2082 may be clamped at both sides of the base support 207, and may be fixed to the base support 207. The decorative ring 201 may be clamped to the bottom side of the first subshell 2081 and the second subshell 2082. The first subshell 2081, the second subshell 2082, and the decorative ring 201 serve as exterior decorations.

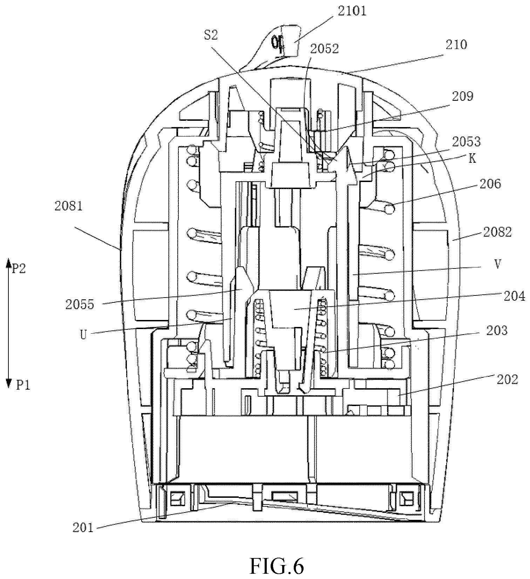

FIG.6 shows a schematic diagram of the structure as shown in FIG.5 installed with an injection switch 210 and a third elastic reset member 209; the insertion device module may also comprise an injection switch 210, and the injection switch 210 may specifically comprise a button glidingly connected to the base support 207 along the injection direction P1; the button may be located in the return direction P2 of the base support 207, specifically inside the chute formed by the inner cylinder 2071 installed on the base support 207 depressed relative to the outer cylinder 2072 along the injection direction P1; a third elastic reset member 209 may be provided between the button and the bottom of the chute, and the third elastic reset member 209 may be a coil spring; the button is abutted to the inclined guide surface S2 (referring to FIG.3a), and the button may move along the injection direction P1; the thrust applied by the button to the inclined guide surface S2 along the injection direction P1 may cause the second buckle 2052 to move along the horizontal direction while pushing the inclined guide surface S2 towards the injection direction P1, thus detaching the second buckle from the clamping hole K, and unlocking the second buckle 2052 and the base support 207. However, it should be understood that the injection switch 210 is not limited to the form of the above-mentioned button, and may also be in the form of a turn button, as long as the injection switch 210 may be operated to unlock the second locking member.

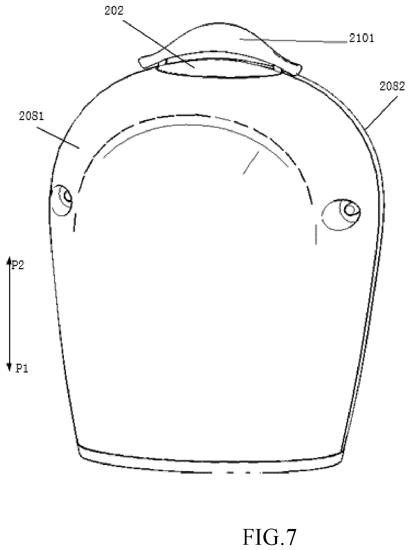

FIG.7 shows a schematic diagram of the external structure of the structure as shown in FIG.6. Referring to FIG.6 and FIG.7, the injection switch 210 is fixedly connected with an anti-touch handle 2101, and the anti-touch handle 2101 may be broken from the injection switch 210. Before the anti-touch handle 2101 is broken, the anti-touch handle 2101 may be clamped on the first subshell 2081 and the second subshell 2082 along the injection direction P1, to prevent from pressing the button due to touch by mistake.

Returning to FIG.4, the main bracket 205 may also comprise a third buckle 2053 connected to the base 2051. When the base support 207 may comprise a main sleeve with an opening facing the injection direction P1, the inner wall of the main sleeve may have a clamping groove V, which may be located in the side wall of the inner cylinder 2071. The clamping slot V may be a groove or a through groove that runs through the side wall of the inner cylinder 2071; when the main bracket 205 is located at the injection station, the clamping positioning surface S3 below the third buckle 2053 may be clamped to the clamping slot V to prevent the main bracket 205 from continuing to slide along the injection direction P1 and detaching from the base support 207.

In FIG.4, each third buckle 2053 may have an integrated structure with a corresponding second buckle 2052, which may face in opposite directions to save space on the base 2051. However, it should be understood that the third buckle 2053 and the second buckle 2052 may also be split structures that are independent of each other, as long as they may achieve their respective functions separately.
The transmitter module 100 will be introduced below in conjunction with the drawings.

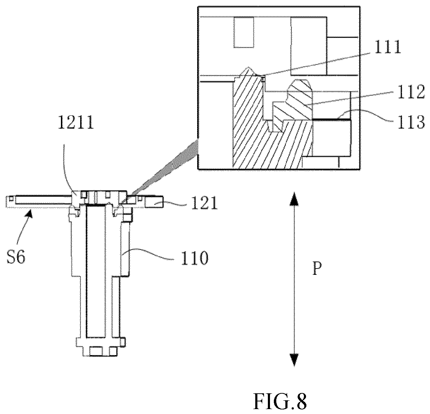

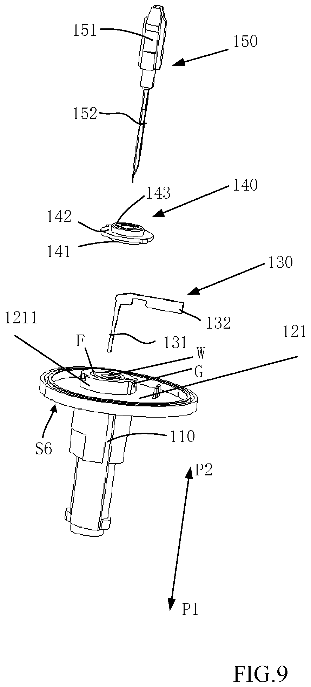

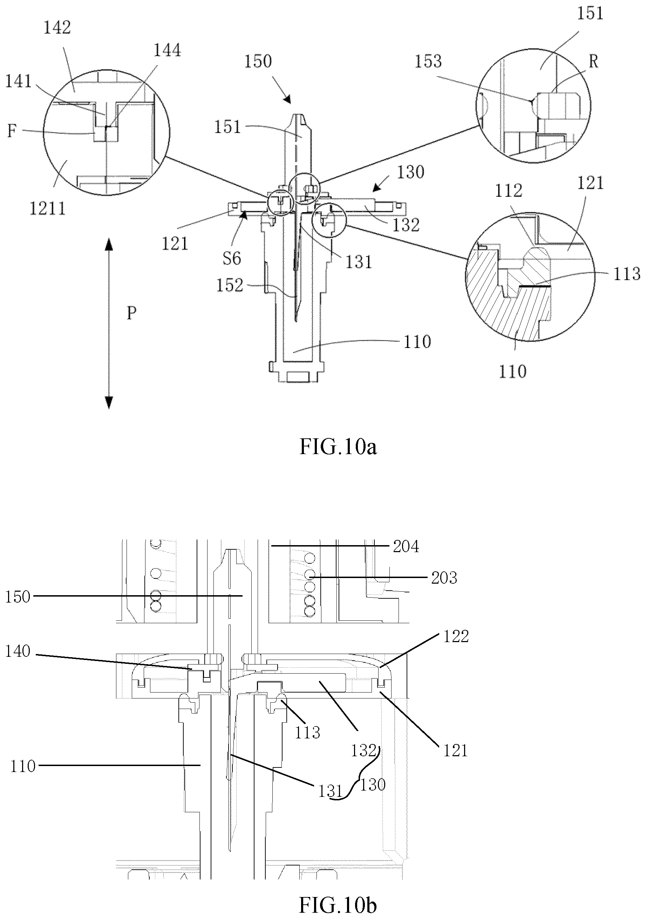

FIG.8 shows a schematic diagram of the structure as shown in FIG.1 assembled with the transmitter bottom shell 121 and the casing 110 of the transmitter module 100, FIG.9 shows a schematic diagram of the structure as shown in FIG.8 assembled with the sensor 130, the sealing bracket 140 and the needle 150, and FIG.10a shows an assembly diagram corresponding to FIG.9. Combining FIG.8, FIG.9, and FIG.10a, the transmitter module 100 may comprise the transmitter bottom shell 121 and the casing 110. The transmitter bottom shell 121 may have an avoidance through-hole W, and the casing 110 may be hermetically connected to the outer surface S6 of the transmitter bottom shell 121. The entire edge of the end of the casing 110 facing the outer surface S6 may have no gap with the outer surface S6, and the corresponding end of the casing 110 may surround the avoidance through-hole W. There are various ways to hermetically connect the casing 110 to the outer surface S6 of the transmitter bottom shell 121. For example, the end of the casing 110 facing the transmitter bottom shell 121 may have an annular connection portion 111 and an annular support platform 113. The annular connection portion 111 may be fixedly connected to the transmitter bottom shell 121, and the annular connection portion 111 mainly plays a fixed connection role. When a needle 150 is needed for injection, only the casing 110 needs to be unscrewed. There may be a first sealing rubber ring 112 between the annular support platform 113 and the transmitter bottom shell 121. The first sealing rubber ring 112 may be squeezed by the annular support platform 113 and the transmitter bottom shell 121 in the axial direction P of the casing 110, thereby achieving the sealing between the annular support platform 113 and the transmitter bottom shell 121. The annular support platform 113 may be located on the periphery of the annular connection portion 111, and the first sealing rubber ring 112 is used to simultaneously protect the connection between the annular connection portion 111 and the transmitter bottom shell 121. There are various ways to fixedly connect the annular connection portion 111 and the transmitter bottom shell 121. While ensuring the fixed connection, the firmness of the connection between the circular connection portion 111 and the transmitter bottom shell 121 should not be too high, so that when using the needle 150 for injection, the casing 110 may be manually unscrewed. For example, the annular connection portion 111 and the transmitter bottom shell 121 may be fixed by ultrasonic welding or glue. Both the annular connection portion 111 and the transmitter bottom shell 121 may be made of plastic materials, such as PC (polycarbonate) and ABS (acrylonitrile butadiene styrene plastic), which may be fixed by ultrasonic welding. The welding quality is stable. With regard to the clamping form, there is no need for complex buckle structures between the casing and the bottom shell, which reduces the requirement for space. However, the fixed connection between the annular connection portion 111 and the transmitter bottom shell 121 does not exclude the form of clamping.

The needle 150 may comprise a needle body 152 and a needle handle 151 connected to one end of the needle body 152. The needle handle 151 may also be made of plastic materials such as PC and ABS. The needle 150 runs through the avoidance through-hole W, and the end away from the needle handle 151 is located inside the casing 110. The sensor 130 may be inserted into the needle body 152, which may be made of metal and have sufficient hardness. The corresponding end of the needle body 152 may be buried in the needle handle 151, and the needle body 152 runs through the avoidance through-hole W, and the end away from the needle handle 151 may be located inside the sleeve 110. However, the sensor 130 may be inserted into the needle body 152, for example, the needle body 152 has a gap extending along its length direction. Apart of the sensor 130 enters the needle body 152 via the above-mentioned gap, so that when the needle 150 is injected, the harder needle body 152 plays a guiding role, helping the softer sensor 130 enter the human body M1 together. When the needle 150 is returned, the needle body 152 is pulled out of the human body M1, and the sensor 130 slides out of the needle body 152 via the gap of the needle body 152 and remains in the human body M1.

The needle handle 151 may be located on the side of the transmitter bottom shell 121 away from the casing 110, and may be hermetically connected to the transmitter bottom shell 121 to block the above-mentioned avoidance through-hole W. It may cooperate with the transmitter bottom shell 121 and the casing 110 to form an enclosed space, providing a sterile environment for the needle body 152 and the sensor 130.

The needle body 152 may be released from the avoidance through-hole W, which limits the needle body 152 throughout the entire process of injection and return, especially during return, preventing the needle body 152 from shaking and tilting and causing bleeding to the user.

There may be various forms of hermetic connection between the needle handle 151 and the transmitter bottom shell 121, as long as it may form the above-mentioned enclosed sterile space.

For example, the transmitter module 100 may also comprise a sealing bracket 140 located between the transmitter bottom shell 121 and the needle handle 151. The sealing bracket 140 may also be made of plastic materials such as PC and ABS. The sealing bracket 140 may comprise an annular support plate 142 and an annular protrusion 141 connected to one side surface of the annular support plate 142. The transmitter bottom shell 121 may have an annular groove F surrounding the avoidance through-hole W. The annular protrusion 141 is located inside the annular groove F and may be filled with sealant 144. The sealant 144 may be applied to the annular groove F first, and then the annular protrusion 141 is placed in the annular groove F. The sealant 144 is filled between the bottom of the annular protrusion 141 and the bottom surface of the annular groove F, thereby forming the sealing between the sealing bracket 140 and the transmitter bottom shell 121. The annular support plate 142 may overlap with the transmitter bottom shell 121, in order to provide stable support for the sealing bracket 140. The sealing bracket 140 may also comprise an annular fence 143 coaxial with the annular protrusion 141, and the annular fence 143 is located on the side of the annular support plate 142 away from the annular protrusion 141 for positioning the needle handle 151.

Specifically, the transmitter bottom shell 121 may have an annular boss 1211 surrounding the avoidance through-hole W, and the annular groove F is provided on the annular boss 1211; the annular support plate 142 may overlap with the annular boss 1211. The sensor 130 may comprise a vertical portion 131 and a horizontal portion 132 connected to one end of the vertical portion 131. The vertical portion 131 is located inside the needle body 152 and is used to collect corresponding indicators inside the human body M1. The horizontal portion 132 may be located on the side of the transmitter bottom shell 121 away from the outer surface S6 and may be fixed to the transmitter bottom shell 121, such as by clamping.

For example, the needle handle 151 has a stepped surface R, and a second sealing rubber ring 153 may be provided between the stepped surface R and the annular boss 1211. The second sealing rubber ring 153 is pressed onto the annular boss 1211 using the stepped surface R to achieve the sealing between the needle handle 151 and the annular boss 1211. Moreover, there may be two contact surfaces between the stepped surface R and the second sealing rubber ring 153, which increases the contact area and improves the sealing effect.

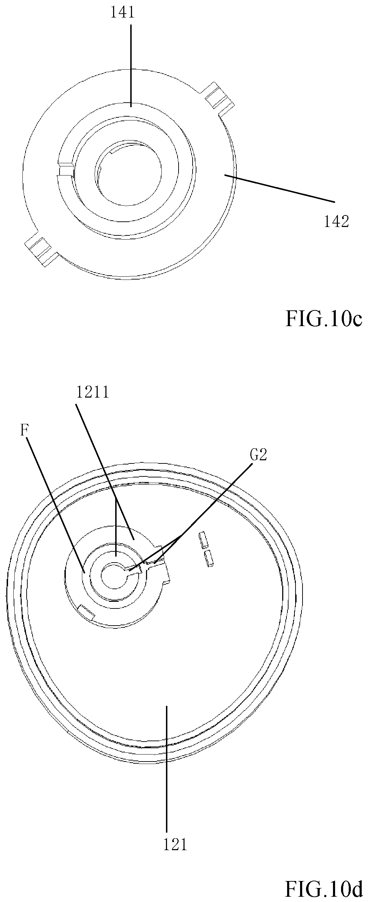

Referring to FIGs. 10b, 10c, and 10d, the annular boss 1211 may be provided with a first slit G1, and the annular protrusion 141 may be provided with a second slit G2 corresponding to the first slit G1. The horizontal portion 132 runs through the first slit G1 and the second slit G2 to extract the signal collected by the vertical portion 131. The first slit G1 and the second slit G2 may also be filled with the above-mentioned sealant 144 to maintain the sealing of the enclosed space and provide good sterile conditions for the needle body 152 and the sensor 130.

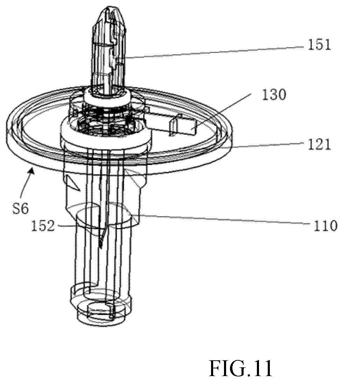

FIG.11 shows a schematic diagram of irradiation sterilization on the structure as shown in FIG.10a. Referring to FIG.11, the transmitter bottom shell 121, needle handle 151, sealing bracket 140 and the casing 110 may be made of transparent materials, such as transparent plastics, to facilitate irradiation sterilization and sterilize the enclosed space where the needle body 152 and the sensor 130 are located, forming a sterile environment.

As mentioned above, since there is no need for the needle handle 151 to pass through the transmitter bottom shell 121 and connect to the casing 110 to form sealing, additional unnecessary return actions are avoided. After the injection is completed, only one return is required.

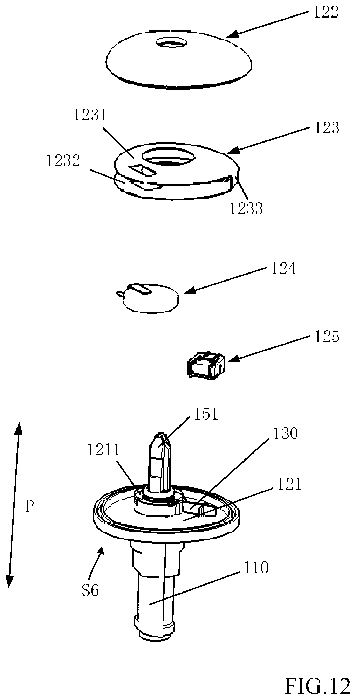

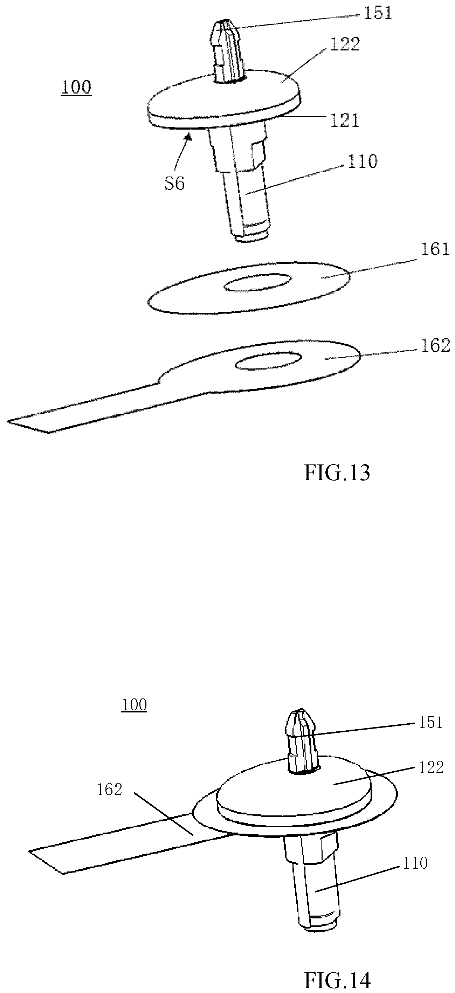

FIG.12 shows an exploded view of the structure as shown in FIG.11 installed with the transmitter top shell 122 and the circuit board 123, and FIG.13 shows an exploded view of the assembly structure installed with the release paper and adhesive in the exploded view as shown in FIG.12. Combining FIG.12 and FIG.13, the transmitter top shell 122 may be located on the side of the transmitter bottom shell 121 away from the casing 110, and encloses an accommodating space with the transmitter bottom shell 121; a circuit board 123 and a connector 125 are further provided in the accommodating space, and the sensor 130 may be connected to the circuit board 123 through the connector 125, in order to transmit the signal collected by the sensor 130 to the circuit board 123, and send the signal processed by relevant devices on the circuit board 123 to video devices such as mobile phones in a wireless manner. A battery 124 is provided on the circuit board 123 to supply power to the sensor 130. In order to increase the usable area of the circuit board 123, the circuit board 123 may comprise a first board portion 1231 and a second board portion 1232. One end of the first board portion 1231 and one end of the second board portion 1232 may be bent and connected through a connection portion 1233. The flexible circuit board may be cut into the above shape, and the first board portion 1231 and the second board portion 1232 may be bent to face each other. The first board portion 1231 and the second board portion 1232 may both be provided with hollow structures for the needle handle 151 to pass through.

The first board portion 1231 may be located between the second board portion 1232 and the transmitter top shell 122, close to the transmitter top shell 122. The end of the first board portion 1231 away from the connection portion 1233 is abutted to the surface of the annular support plate 142 facing the transmitter bottom shell 121, and the annular support plate 142 is used to limit the first board portion 1231.

FIG.14 shows an assembly diagram corresponding to FIG.13. Referring to FIG.13 and FIG.14, the transmitter bottom shell 121, transmitter top shell 122, and the part therebetween may form the transmitter N. The outer surface S6 of the transmitter bottom shell 121 may have adhesive 161, and the surface of adhesive 161 may have release paper 162. When the needle body 152 of the needle 150 is injected into the human body M1, the transmitter bottom shell 121 may be adhered to the skin surface of the human body M1 through the adhesive 161, and the transmitter bottom shell 121 may be adhered to the skin surface through the adhesive 161 to send the signal collected by the sensor 130 in a wireless manner.

A brief explanation will be given below to the assembly process of the transmitter module 100 and the insertion device module 200.

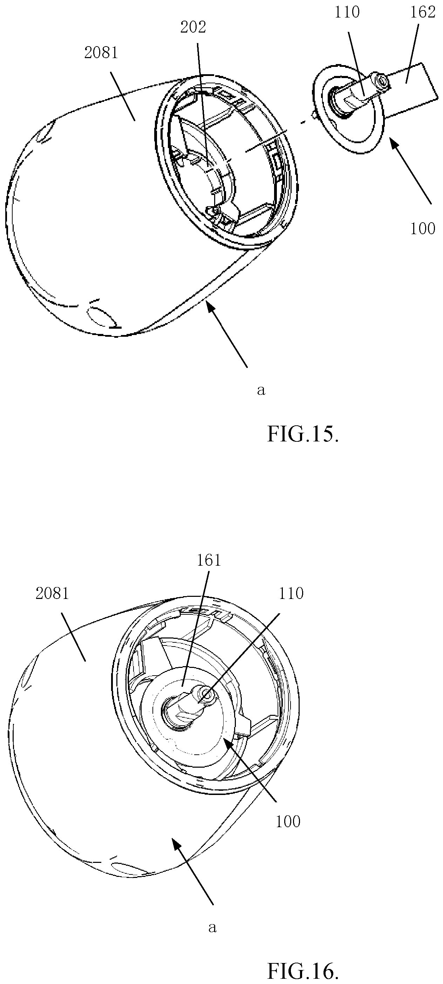

FIG.15 shows a schematic diagram before installing the transmitter module 100 as shown in FIG.14 into the main body a, and FIG.16 shows a schematic diagram after installing the transmitter module 100 as shown in FIG.14 into the main body a. Combining FIG.15 and FIG.16, the transmitter module 100 is installed in the main body a, and the needle handle 151 is clamped to the hoop 2043, with the release paper 162 torn off to expose the adhesive 161. The transmitter top shell 122 and transmitter bottom shell 121 may be pre-fixed on the transmitter bracket 202, for example, by a buckle, but the pre-fixing force is less than the bonding force between the adhesive 161 and the human body M1 skin. When the main body a injects the transmitter module 100 into the human body M1, the transmitter bottom shell 121 is adhered to the surface of the human body M1 skin through the adhesive 161. When the main body a is removed, the transmitter top shell 122 and the transmitter bottom shell 121 detach from the transmitter bracket 202 and remain on the surface of the human body M1 skin.

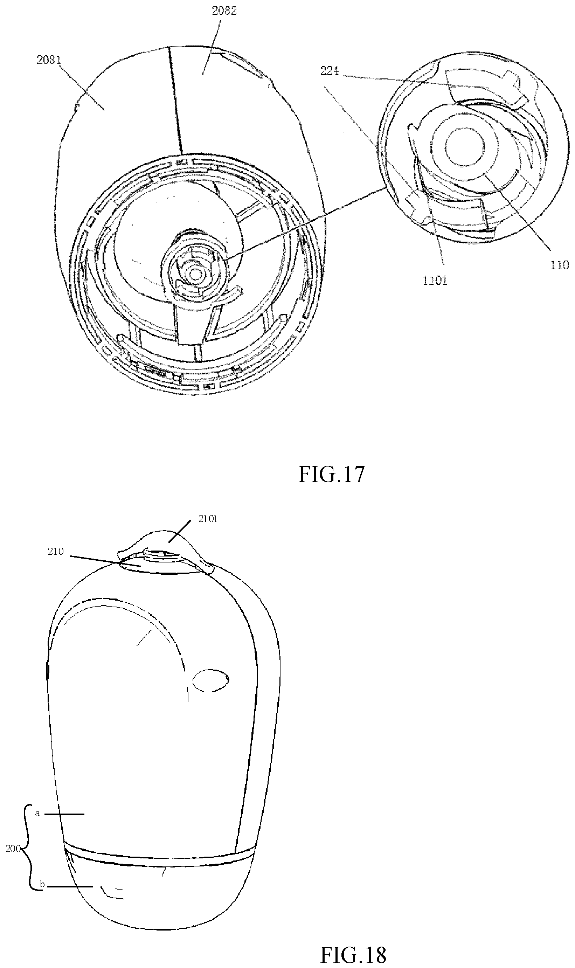

FIG.17 shows a sectional view of the structure as shown in FIG.1 after fixing the bottom shell b to the bottom side of the main body a. Combining FIG.1 and FIG.17, the bottom shell b may comprise an arc-shaped shell body 221, and there may be two elastic buckles 224 in the middle of the shell body 221. The forms of the elastic buckles 224 may be arc-shaped spring pieces, and the elastic buckles 224 are fixedly connected to the shell body 221 through a middle support skeleton 222. The outer surface of the sleeve 110 may have ratchet teeth 1101. After the elastic buckle 224 is sleeved outside of the casing 110, FIG.18 shows a schematic diagram of the appearance of the human body indicator monitoring device corresponding to FIG.17. Referring to FIG.18, a complete human body indicator monitoring device is formed. The bottom shell b is rotated in one direction, and the elastic buckle 224 will be pushed open by the ratchet teeth 1101. The bottom shell b is rotated in an opposite direction, and the ratchet teeth 1101 and the elastic buckle 224 may get stuck. Continuing to rotate the bottom shell b may unscrew the casing 110 and expose the needle body 152.

The use process of the human body indicator monitoring device provided in the embodiment of the present application will be introduced below.

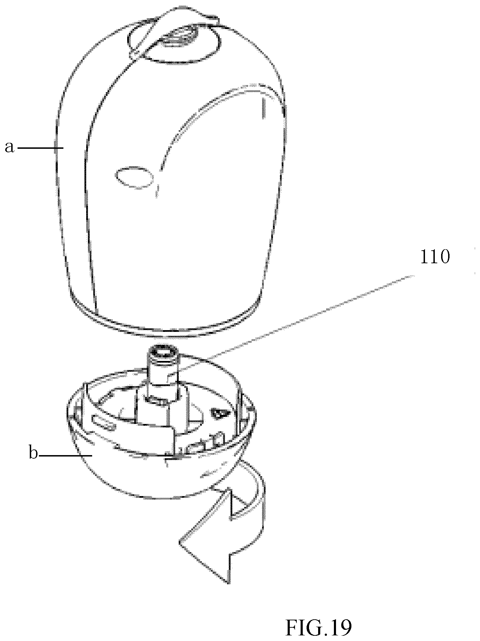

FIG.19 shows a schematic diagram of the human body indicator monitoring device provided in the embodiment of the present application with the bottom shell b unscrewed. Referring to FIG.19, the bottom shell b is rotated, and the elastic buckle 224 is used to clamp the ratchet teeth 1101. The casing 110 is unscrewed from the transmitter bottom shell 121, leaving the casing 110 in the bottom shell b.

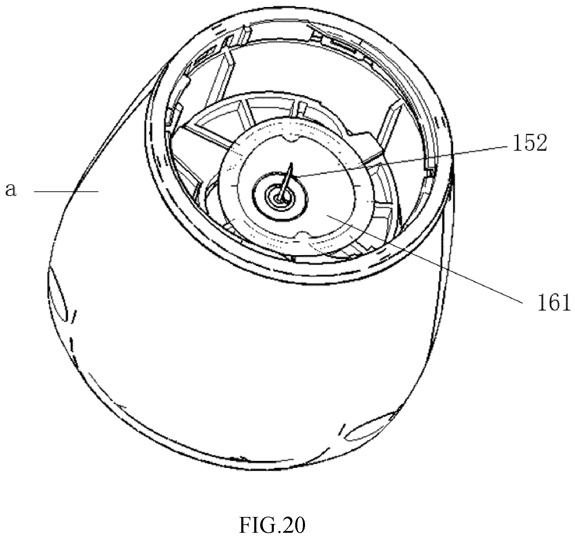

FIG.20 shows a schematic diagram of the structure as shown in FIG.19 with the bottom shell b removed. Referring to FIG.20, the needle body 152 is exposed, and the adhesive 161 is exposed.

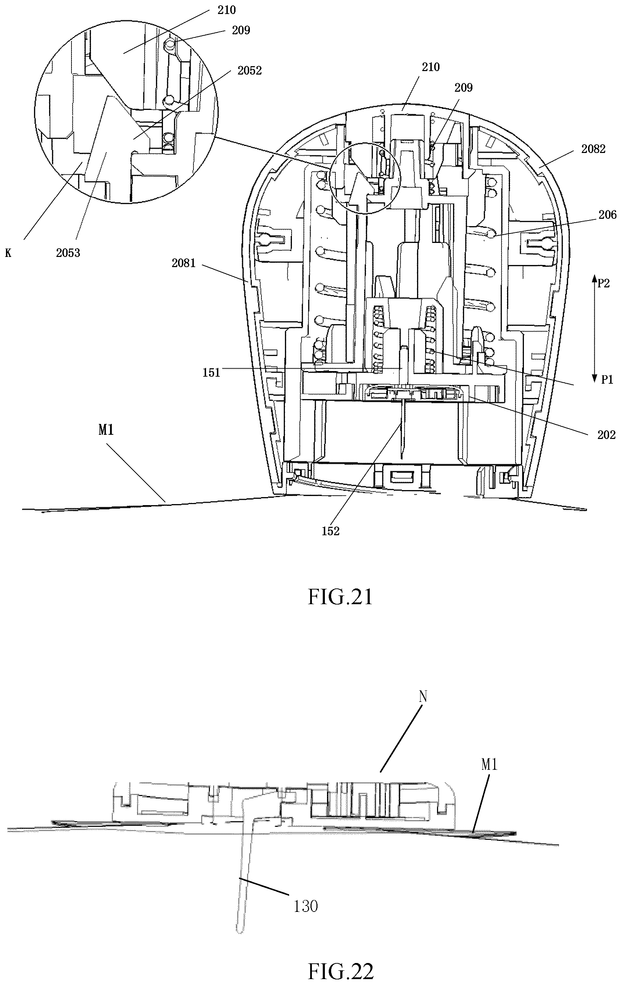

FIG.21 shows a schematic diagram of the structure as shown in FIG.20 after breaking the anti-touch handle 2101. Referring to FIG.21, after pressing the button (injection switch 210), the second buckle 2052 is detached from the clamping hole K, the main bracket 205 moves along the injection direction P1, and the needle body 152 enters the human body M1. And when the main bracket 205 reaches the unlocking station, at least part of the first buckle 2055 enters the avoidance groove U, releasing the locking on the needle bracket 204. The needle bracket 204 with the needle 150 returns along the return direction P2, pulling out the needle body 152 from the human body M1. FIG.22 shows a state diagram of the sensor 130 and the transmitter N in FIG.21 when working in a human body M1. Referring to FIG.22, the sensor 130 slides out of the needle body 152 through the gap of the needle body 152 and remains inside the human body M1, while the transmitter N remains on the surface of the human body M1 through the adhesive 161.

Continuing to refer to FIGs. 19 to 21, based on the same inventive concept, the embodiments of the present application also provide a method of using the human body indicator monitoring device as provided in the above embodiments, and the insertion device module 200 comprises: a base support 207, a main bracket 205, a needle bracket 204, a first elastic reset member 206 and a second elastic reset member 203, wherein the main bracket 205 comprises a base 2051 and a first locking member 2055 connected to the base 2051;

The method may comprise:
taking the insertion device module 200 closer to the human body M1;
releasing the limit between the main bracket 205 and the base support 207,
wherein the first elastic reset member 206 drives the main bracket 205 to move from an initial station to an injection station via an unlocking station along an injection direction P1; when the main bracket 205 is located at the initial station, the first locking member 2055 is clamped between the needle bracket 204 and the base support 207, and locked to the needle bracket 204; when the main bracket 205 moves to the unlocking station, at least part of the first locking member 2055 is located inside an avoidance groove U of the base support 207 to unlock the needle bracket 204;
the first elastic reset member 206 continues to drive the main bracket 205 to move to the injection station, so that the needle 150 and the sensor 130 are injected into a human body M1, and the second elastic reset member 203 drives the needle bracket 204 to return from an original station to a return station along a return direction P2, so as to pull the needle 150 out of the human body M1, wherein the return direction P2 is opposite to the injection direction P1; and
removing the insertion device module 200.

After the step of pulling the needle 150 out of the human body M1 and leaving the sensor 130 inside the human body M1, the method may also comprise:
the sensor 130 is in signal connection to a transmitter, and a signal collected by the sensor 130 is transmitted to an additional device via the transmitter in a wireless manner; or, the sensor is in direct signal connection to the additional device, or, the transmitter is in signal connection to the sensor 130, and a signal collected by the sensor 130 is transmitted to the additional device via the transmitter.

The beneficial effects of this method may refer to the effect analysis of the human body indicator monitoring device.

It will be apparent to those skilled in the art that various amendments and variations may be made to the embodiment of the present invention without departing from the spirit and scope of the present invention. In this way, if these amendments and variations of the present invention are within the ranges of the claims of the present invention and equivalent technologies thereof, the present invention has also intended to contain those amendments and modifications.

## Claims

1. A transmitter module (100), wherein the module comprises a transmitter bottom shell (121), a casing (110), a needle (150), and a sensor (130), wherein the transmitter bottom shell (121) has an avoidance through-hole (W); the casing (110) is hermetically connected to an outer surface of the transmitter bottom shell (121), and the casing (110) surrounds the avoidance through-hole (W);
wherein the needle (150) comprises a needle body (152) and a needle handle (151) connected to one end of the needle body (152); the needle handle (151) is located on the side of the transmitter bottom shell (121) away from the casing (110) and is hermetically connected to the transmitter bottom shell (121) to block the avoidance through-hole (W);
wherein the needle body (152) runs through the avoidance through-hole (W), and the end away from the needle handle (151) is located inside the casing (110), with the sensor (130) inserted into the needle body (152).

2. The transmitter module (100) according to claim 1, wherein the end of the casing (110) facing the transmitter bottom shell (121) has an annular connection portion (111) and an annular support platform (113); the annular connection portion (111) is fixedly connected to the transmitter bottom shell (121), and a first sealing rubber ring (112) is provided between the annular support platform (113) and the transmitter bottom shell (121).

3. The transmitter module (100) according to claim 2, wherein the annular connection portion (111) and the transmitter bottom shell (121) are fixed by ultrasonic welding or glue.

4. The transmitter module (100) according to claim 1, wherein the transmitter module (100) further comprises a sealing bracket (140) located between the transmitter bottom shell (121) and the needle handle (151);
wherein the sealing bracket (140) comprises an annular support plate (142) and an annular protrusion (141) connected to one side surface of the annular support plate (142), and the transmitter bottom shell (121) has an annular groove (F) surrounding the avoidance through-hole (W); the annular protrusion (141) is located inside the annular groove (F), and the annular groove (F) is filled with sealant;
wherein the annular support plate (142) overlaps with the transmitter bottom shell (121).

5. The transmitter module (100) according to claim 4, wherein the transmitter bottom shell (121) has an annular boss (1211) surrounding the avoidance through-hole (W), and the annular groove (F) is provided on the annular boss (1211);
wherein the sensor (130) comprises a vertical portion (131) located inside the needle body (152) and a horizontal portion (132) connected to one end of the vertical portion (131); the annular boss (1211) is provided with a first slit (G1), and the annular protrusion (141) is provided with a second slit (G2) corresponding to the first slit (G1); the horizontal portion (132) is located on the transmitter bottom shell (121), and runs through the first slit (G1) and the second slit (G2); the first slit (G1) and the second slit (G2) are filled with the sealant.

6. The transmitter module (100) according to claim 5, wherein the needle handle (151) has a stepped surface (R), and a second sealing rubber ring (153) is provided between the stepped surface (R) and the annular boss (1211).

7. The transmitter module (100) according to any of claims 4-6, wherein the needle handle (151), the sealing bracket (140), the transmitter bottom shell (121) and the casing (110) are all made of transparent materials.

8. The transmitter module (100) according to any of claims 4-6, wherein the transmitter module (100) further comprises a transmitter top shell (122), and the transmitter top shell (122) is located on the side of the transmitter bottom shell (121) away from the casing (110), and encloses an accommodating space with the transmitter bottom shell (121);
wherein a circuit board (123) and a connector (125) are further provided in the accommodating space, and the sensor (130) is connected to the circuit board (123) through the connector (125);
wherein the circuit board (123) comprises a first board portion (1231) and a second board portion (1232); one end of the first board portion (1231) and one end of the second board portion (1232) are bent and connected by a connection portion (1233); the first board portion (1231) is located between the second board portion (1232) and the transmitter top shell (122); the end of the first board portion (1231) away from the connecting portion (1233) is abutted to the surface of the annular support plate (142) facing the transmitter bottom shell (121).

9. The transmitter module (100) according to claim 1, wherein the needle body (152) is in contact with the inner wall of the avoidance through-hole (W).

10. The transmitter module (100) according to claim 1, wherein the outer surface of the transmitter bottom shell (121) is provided with adhesive (161).

11. A human body indicator monitoring device, wherein the device comprises an insertion device module (200) and the transmitter module (100) according to any of claims 1-10, wherein the transmitter module (100) is installed on the insertion device module (200), and the insertion device module (200) is used to inject a needle (150) and sensor (130) of the transmitter module (100) into a human body (M1), and used to return the needle (150).

12. A method of using the human body indicator monitoring device according to claim 11, wherein the insertion device module (200) comprises: a base support (207), a main bracket (205), a needle bracket (204), a first elastic reset member (206) and a second elastic reset member (203), wherein the main bracket (205) comprises a base (2051) and a first locking member (2055) connected to the base (2051);
wherein the method comprises:
taking the insertion device module (200) closer to the human body (M1);
releasing the limit between the main bracket (205) and the base support (207),
wherein the first elastic reset member (206) drives the main bracket (205) to move from an initial station to an injection station via an unlocking station along an injection direction (P1); when the main bracket (205) is located at the initial station, the first locking member (2055) is clamped between the needle bracket (204) and the base support (207), and locked to the needle bracket (204); when the main bracket (205) moves to the unlocking station, at least part of the first locking member (2055) is located inside an avoidance groove (U) of the base support (207) to unlock the needle bracket (204);
wherein the first elastic reset member (206) continues to drive the main bracket (205) to move to the injection station, so that the needle (150) and the sensor (130) are injected into a human body (M1), and the second elastic reset member (203) drives the needle bracket (204) to return from an original station to a return station along a return direction (P2), so as to pull the needle (150) out of the human body (M1), wherein the return direction (P2) is opposite to the injection direction (P1); and
removing the insertion device module (200).

13. The method according to claim 12, wherein the method, after pulling out the needle (150) from the human body (M1) and leaving the sensor (130) inside the human body (M1), further comprises:
the sensor (130) is in signal connection to a transmitter, and a signal collected by the sensor (130) is transmitted to an additional device via the transmitter in a wireless manner; or, the sensor is in direct signal connection to the additional device, or, the transmitter is in signal connection to the sensor (130), and a signal collected by the sensor (130) is transmitted to the additional device via the transmitter.
